(19) Europäisches Patentamt
European Patent Office
Office européen des brevets

(11) **EP 2 041 155 B1**

(12) **EUROPEAN PATENT SPECIFICATION**

(45) Date of publication and mention
of the grant of the patent:
**08.01.2020 Bulletin 2020/02**

(51) Int Cl.:
**C07K 1/34** (2006.01)     **A61L 2/00** (2006.01)
**B01D 61/02** (2006.01)

(21) Application number: **07766797.0**

(22) Date of filing: **25.06.2007**

(86) International application number:
**PCT/IL2007/000764**

(87) International publication number:
**WO 2008/001353 (03.01.2008 Gazette 2008/01)**

(54) **VIRUS REMOVAL BY NANOFILTRATION**

ENTFERNUNG VON VIREN DURCH NANOFILTRATION

ÉLIMINATION DE VIRUS PAR NANOFILTRATION

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **26.06.2006 US 816393 P**

(43) Date of publication of application:
**01.04.2009 Bulletin 2009/14**

(73) Proprietor: **Omrix Biopharmaceuticals Inc.
Somerville, NJ 08876 (US)**

(72) Inventors:
• **NUR, Israel
Nes-ziona (IL)**
• **Mintz, Roni
79515 (IL)**

(74) Representative: **Grünecker Patent- und
Rechtsanwälte
PartG mbB
Leopoldstraße 4
80802 München (DE)**

(56) References cited:
**EP-A- 1 348 445     WO-A-98/37086
WO-A-99/19343**

• **DILEO A J ET AL: "SIZE EXCLUSION REMOVAL OF MODEL MAMMALIAN VIRUSES USING A UNIQUE MEMBRANE SYSTEM, PART II: MODULE QUALIFICATION AND PROCESS SIMULATION" BIOLOGICALS, ACADEMIC PRESS LTD., LONDON, GB, vol. 21, no. 3, 1 September 1993 (1993-09-01), pages 287-296, XP000670924 ISSN: 1045-1056 cited in the application**

**EP 2 041 155 B1**

**Description**

**FIELD OF THE INVENTION**

This invention relates to purification of protein solutions. In particular, the invention relates to a method of enhancing the removal of infective particles from a thrombin solution by nanofiltration.

**BACKGROUND OF THE INVENTION**

Human blood is the source of a wide range of medicinal products used for the treatment of acquired and/or congenital hematological disorders and life threatening diseases. Examples for such products include immunoglobulin, Factor VIII, albumin, $\alpha$1 anti trypsin, anti thrombin III, Factor IX, Factor XI, PCC's (Prothrombinase complex concentrates), fibrinogen and thrombin.

Since human blood, the raw material of these products, may contain infectious agents which are responsible for the transmission of serious diseases, the sterility of the products is of major concern. Thus, great efforts are invested in the inactivation and /or removal of infectious agents such as hepatitis viruses, human immunodeficiency virus (HIV), and transmissible spongiform encephalopathies (TSE), which may be present in these preparations.

Lipid coated viruses can be effectively inactivated by treating the in-process material with organic solvents and surfactants, as describe for example in EP 0131740, and US 4481189. However, since this solvent detergent process has no significant effect on non-enveloped viruses nor on TSE agent, other inactivation methods such as pasteurization, gamma or UVC (< 280 nm) irradiation, or nanofiltration should be applied.

Among these inactivation methods, nanofiltration is the milder one. There are two operational methods for nanofiltration: dead-end and tangential flow.

(1) Dead end - where fluid is directed towards the membrane under an applied pressure, particles larger from the membrane pore size will accumulate at the membrane surface while smaller molecule will pass through.
(2) Tangential flow nanofiltration (TFNF)-where the fluid is pumped tangentially along the surface of the membrane establishing a pressure differential across the membrane, which causes particles smaller than the pore size to pass through the membrane, while larger particles continue to flow across the membrane by tangential flow. The tangential flow prevents retained molecule buildup on the membrane surface.

DiLeo, A.J., et al (1992) Biotechnology 10:182-188, describes the removal of virus particles of small diameter (<30nm), such as the $\varphi$X174 phage, from a solution using the Viresolve/70™ membrane. In order to investigate the extent to which particle adsorption contributes to the removal of virus particles, 2.5 mg/ml human serum albumin (HSA) was added to the viral solution to competitively reduce viral adsorption to the membrane. It was found that the virus retention increased by 0.5-0.7 logs in the presence of HSA protein. The authors state that these results indicate that the particle retention properties of the membrane are primarily a result of the sieving properties of the membrane, and the increase in virus retention is most likely the result of protein concentration polarization at the membrane surface.

DiLeo, A.J., et al (1993) Biologicals 21:275-286, describes further experiments carried out with the Viresolve/70™ membrane. It was once again found that the presence of protein increases the retention value of the model viruses in accordance with concentration polarization in which polarized protein adds an additional resistance to virus passage through the membrane.

Hoffer, L. et al (1995) J. Chromatography 669:187-196, describes the purification of Factor IX from human plasma and virus removal using the Viresolve/70™ membrane. The smallest virus measured was canine parvovirus (16-18 nm diameter), which was removed with a log reduction value (LRV) of 5.2. The average % recovery of Factor IX was 83±9%.

WO 96/00237 (Winge) discloses a method of virus-filtering a solution that contains macromolecules, primarily proteins, polysaccharides and polypeptides, by increasing the total salt content of the solution from about 0.2 M upto saturation of the solution with the salt concerned. The method reduces the residence time and the extent to which the solution need to be diluted, and optimizes the yield when virus-filtering. The disclosed method facilitates virus filtration with the aid of the so-called "dead-end" technique, which affords several process and economic advantages in comparison with the tangential virus-filtering technique normally used.

US 6,096,872 (Van Holten, et al) discloses a method for nanofiltration of the anti-D immunoglobulin in high ionic strength buffer and with an excipient such as polysorbate 80™. Additional steps include diafiltration to concentrate the anti-D protein and reduce the concentration of excipient present. In particular, the method comprises the following steps: (a) fractionating human plasma in alcohol; (b) resuspending the resulting Precipitate II; (c) admixing the resuspended Precipitate II with a high ionic strength buffer containing an excipient; and (d) performing nanofiltration on the immunoglobulin.

US 6,773,600 (Rosenblatt, et al) discloses a method for purifying a proteinacious material such as an immunoglobulin for removal of contaminants such as viral pathogens, comprising the steps of:

(a) admixing the proteinaceous material with:

(i) a low pH, low conductivity buffer solution formulated to reduce the pH between 5.0 and 6.0, and to achieve an ionic strength of less than 30 mS/cm;
(ii) a non-ionic surfactant; and
(iii) a clathrate modifier; and

(b) performing nanofiltration on the proteinaceous material to obtain a purified material substantially free of viral particles.

Preferably, the clathrate modifier is a polyol sugar or sugar alcohol having from 4 to 8 hydroxyl groups.

## SUMMARY OF THE INVENTION

The present invention is defined by the claims. Any subject matter falling outside the scope of the claims is provided for information only.

In one aspect of the invention, there is provided a method for enhancing infective particle removal from a thrombin solution. The method comprises:

**(a)** adding a macromolecule to the protein solution; and
**(b)** passing the solution through a nanofilter,

thereby obtaining a protein solution substantially purified from the infective particles, wherein the macromolecule is not a non-ionic surfactant.

The application additionally discloses a method for enhancing infective particle removal from a protein solution by nanofiltration, the method comprising adding a macromolecule to the solution prior to and/or during nanofiltration.

The term "infective particle" refers to a microscopic particle, such as a virus or a prion, that can infect or propagate in cells of a biological organism. In one embodiment, the infective particles are viral particles. The infective particles to be removed are in the size range of 15 to 80nm. In another embodiment, the infective particles are larger than 80nm.

The term "protein solutions" refer to a homogeneous mixture composed of one or more proteins, dissolved in another substance. In one embodiment, the solution containing the protein originates from whole blood. In another embodiment, the solution is plasma. Other possible sources of protein include animal proteins, and recombinant proteins produced in cell culture or in transgenic animals.

The proteins which may be purified by the method of the present invention include all therapeutically useful proteins which may harbor infective particles and which are filtered with an infective particle removal filter. In one embodiment, the proteins have a molecular weight of less than 180 kDa. In another embodiment, the proteins have a molecular weight of less than 160 kDa. In a further embodiment, the proteins have a molecular weight of less than 150 kDa, or less than 140 kDa.

These proteins include but are not limited to:

Coagulation factors: e.g. Factor IX/IXa, Factor VIII; prothrombin/thrombin, Factor VII/VIIa, Factor X/Xa, Factor XI/XIa, Factor XII/XIIa, prekallikrein, HK, plasminogen, urokinase, protein c inhibitor;
Anti-coagulation factors or subunits thereof: e.g. protein C, protein S, anti-thrombin III, heparin cofactor II, alpha2 anti-plasmin, protein Z;
Growth factors: e.g. insulin-like growth factor 1 (IGF-1), granulocyte colony-stimulating factor (G-CSF), granulocyte-macrophage colony-stimulating factor (GM-CSF);
Neurotropic factors, e.g. nerve growth factor (NGF), glial cell line-derived neurotrophic factor (GDNF);
Hormones: e.g. erythropoietin, growth hormone;
Interferons and interleukins: e.g. interferon alpha, beta and gamma, interleukin 1, 2, 3, and 7;
Recombinant proteins: e.g. recombinant coagulation factors or anti-coagulation factors, recombinant growth hormone.

In one embodiment, the protein is a polar protein. A polar protein refers to a hydrophilic molecule.

The protein yield using the method of the invention is advantageously high and may be at least about 59, 65, 71, 72, 75, 80, 81, 84, 85, 87, 93, 94, 96 or 100%. The average protein recovery using the method of the invention was found to be in the range of 75-93%.

The term "substantially purified" refers to the efficiency of infective particle removal. In one embodiment, the efficiency of infective particle removal is above 4 LRV (log reduction value), or about 5, 6, 7, or 8 LRV.

The definition of the term "macromolecule" implies conventional polymers and biopolymers as well as non-polymeric molecules with a large molecular mass. A macromolecule of the invention is not a non-ionic surfactant. A macromolecule which may be used in the method of the invention should have, in one embodiment, at least one of the following properties, in another embodiment two of the properties, and in a further embodiment all of the following properties:

- Biocompatible - non toxic;
- does not interact with the protein undergoing purification;
- is water soluble.

The macromolecule according to the invention may be a polymer of at least 3 monomers of sugars, amino acids, glycols, alcohols, lipids or phospholipids. In one embodiment, the macromolecule is a polymer of at least 6 monomers. In another embodiment, the macromolecule is a polymer of at least 9 monomers. In a further embodiment, the macromolecule is a polymer of at least 12 monomers. Examples of macromolecules according to the invention include but are not limited to proteins (e.g. albumin, gelatin), polysaccharides (e.g. dextran, chitosan, carrageen), polymer of sugar alcohols (e.g. mannitol polymer), phospholipids, polyvinylpyrrolidone (PVP), polyethylene glycol (PEG), cellulose derivatives, vinyl polymers and polyglycols. The macromolecule does not include a polyol sugar or sugar alcohol having from 4 to 8 hydroxyl groups.

In one embodiment of the invention, the macromolecule is albumin. In another embodiment of the invention, the macromolecule is dextran. The dextran molecules can be of different molecular weights, for example, in a range of 5-80kDa or about 5, 25 and 80 kDa.

In one embodiment, the macromolecules may be added within a concentration range of more than 0.01 mg/ml and up to less than or equal to 2 mg/ml. In another embodiment, the macromolecule is added within a concentration range of more than 0.15 $\mu$M to less than or equal to 31 $\mu$M. In a further embodiment, the macromolecule is added at a concentration that is lower than the concentration of the protein solution.

Nanofiltration relates to a pressure driven separation process, which may take place on a selective separation layer formed by a semipermeable membrane. The driving force of the separation process is the pressure difference between the feed (retentate) and the filtrate (permeate) side at the separation layer of the membrane.

In one embodiment of the invention, the nanofilter which may be used in the method of the invention may have a particle size cutoff of >$10^4$ Dalton. In another embodiment, the cutoff may be > $10^5$ Dalton. Another example is a cutoff in the range of $10^4$ Dalton and $3 \times 10^5$ Dalton. In one embodiment, the nanofilter is a Viresolve/70™ or Viresolve/180™ membrane.

The solution is passed through the nanofilter in accordance with standard filtration procedures. For example, the solution may be passed through the nanofilter by either tangential flow nanofiltration (TFNF) or by dead-end nanofiltration. Stabilizers such as mannitol and salts may also be added to the protein solution.

A further disclosure of the application is a protein solution purified from infective particles by the method of the invention.

## BRIEF DESCRIPTION OF THE DRAWINGS

In order to understand the invention and to see how it may be carried out in practice, exemplary embodiments will now be described, by way of non-limiting example only, with reference to the accompanying drawings, in which:

**Fig. 1** is a schematic diagram illustrating the assembly of the Viresolve 70™ Membrane System; and

**Fig. 2** is a graph showing the effect of HSA concentration on MVMp removal (LRV) through Viresolve 70 filtration.

The disclosure of applications, patents and publications, cited above or below, is hereby incorporated in their entirety by reference.

The following examples are illustrative but not limiting.

## DETAILED DESCRIPTION OF EXEMPLARY EMBODIMENTS

### Material and Methods

### Viruses and Cells

### Cells

**A9** (ATCC- CCL-1.4) mouse fibroblast cell line was routinely maintained in Dulbecco's modified Eagle medium (DMEM) supplemented with 2mM L-Glutamine, 1% penicillin-streptomycin-amphotericin B solution, 0.1% gentamycin sulfate solution, and 5% fetal calf serum (FCS) (Biological Industries).

**FrhK-4** (Wallace R.E et.al., In vitro 8:333-341, 1973) continuous cell line from the kidney of a rhesus monkey, grown in DMEM with 4.5g/l glucose 110mg/l sodium pyruvate and 1.1 g/l sodium bi carbonate supplemented with 4mM glutamine 1% non-essential amino-acids and 10% FCS.

### Viruses

**Minute parvovirus of mice (MVM(p))** was kindly provided by Professor Jacov Tal (Ben-Gurion University) and was propagated in A9 cells. Virus production was performed as described previously (Tattersall P, Bratton J. (1983)) Briefly, A9 cells were infected with MVMp at 0.1-1 multiplicity of infection (MOI). Once an extensive cytopathic effect was observed, e.g. about 30% of the cells become detached, cells and medium were collected and centrifuged at 1000g for 10 minutes at 4°C. The cell pellet was then resupended in TNE buffer (Tris-HCl, 50mM pH 7.2, NaCl 150mM, EDTA 50mM) and recentrifuged at 1000g as above. The process was repeated 3 times. At the end of the last centrifugation step, the cell pellet was resuspended in TE buffer (Tris-HCl 50mM pH8.7, EDTA 0.5mM) and then subjected to three cycles of freezing in liquid nitrogen and thawing at room temperature. To purify the virus, the disrupted cells were subjected to centrifugation at 20,000g for 10 minutes at 4°C. The supernatant containing the virus was aliquoted and stored at <-70°C. Prior to use virus stock was subjected to 0.2$\mu$m filtration (Minisart, Sartorius) followed by 0.1$\mu$m filtration (Durapore,Millipore).

**Hepatitis A virus (HAV)** (HM175) (Cooper P et al., 1978) was produced in the supernatant of infected FrhK-4 cells. After clarification at 3500rpm for 15 minutes (Sigma 3K2), the cells were collected and resuspended in PBS and sonicated-clarified at 2500 rpm for 10 minutes. The supernatant was subsequently aliquoted and stored at <-70°C. The supernatant from the first centrifugation was concentrated by ultracentrifugation at 19000rpm for 7 hours at 4°C (Beckman R19). The pellet was resuspended in PBS aliquoted and stored at <-70°C.

### Virus titration and log reduction value (LRV) evaluation

MVM(p) virus stock and all study samples titres were determined by the $TCID_{50}$ assay. Briefly, virus/sample dilutions were prepared by a series of two-fold dilutions in a 96 well plate using A9 cells growth medium as diluent (final volume of 50$\mu$l). To each well, 100$\mu$l of A9 cells ($5 \times 10^3$) were added and plates were incubated at 37°C for 7-10 days. Samples containing a high titter of MVM(p) virus, were pre-diluted in order to be quantified using the above assay. Each well was examined for infectivity and the titter was determined and expressed as 50% tissue culture infective dose per millilitre ($TCID_{50}$/ml) using the Spearman-Kärber formula. HAV samples titters were determined as described above with minor changes; FrhK-4 cells were used at a concentration of $1 \times 10^4$ cells per well and infectivity was determined at about 10-12 days post infection.

In the case that no infectivity is detected in the sample tested, either a larger volume of sample is assayed at the minimal non cytotoxic dilution, or a large amount of the sample is subjected to ultracentrifugation (Beckman R19 rotor at 19000rpm for 15 hours at 4°C) and the entire volume is tested for infectivity.

LRV was calculated according to the following formula:

$$LRV = \log (\text{virus titter in the first sample} \times \text{sample volume}) - \log (\text{virus titter in the second sample} \times \text{sample volume})$$

### Spiking

The samples used in the Viresolve 70™ filtration study, were spiked with MVMp or HAV to reach a final concentration higher than $1 \times 10^5$ $TCID_{50}$ units/ml. The ratio of spiking did not exceed 10%. The spiked solutions were filtered through a 0.2μm filter (Millipak 40, Millipore) prior to Viresolve 70™ filtration.

**Thrombin quantitation**

Quantitation of thrombin activity was performed according to the European Pharmacopeia Assay (0903/1997) procedure with slight modification. A calibration curve of (4-10 IU/ml) was prepared by mixing thrombin standard with a fibrinogen solution of 0.1% fibrinogen (Enzyme Research Laboratories, Ltd.) content. The thrombin concentration of the sample was calculated from the calibration curve and multiplied by the dilution factor.

**Determination of total protein concentration**

Protein concentration was determined using the Biuret method (Doumas et al, 1981).
Previous to Biuret reagent addition, samples were subjected to acetone precipitation. Samples and standard were diluted 2.5-fold with acetone, incubated for 5 minutes and then centrifuged for 5 minutes at 20,000g. The pellet was resuspended with 0.1ml of saline by vigorous vortexing and 0.9ml of Total Protein Reagent (Sigma) was added to each sample. After incubation for 15 minutes at room temperature, samples and standard absorbencies were measured at 540nm using a spectrophotometer. Protein concentration was calculated by comparing sample results to standard results.

**Thrombin in-process samples**

Thrombin is routinely produced from cryopoor plasma at the Omrix production facility (Tel Aviv, Israel). During the production process the product is subjected to a solvent detergent (1% TnBP/ 1% Triton-XlOO) treatment to eliminate enveloped viruses. The solvent and detergent are then removed by chromatography while the product is subsequently recovered by elution. To increase product stability, mannitol is added to a final concentration of 2%. HSA is added to a final concentration of 0.2%. The product is then subjected to Viresolve70™ filtration at room temperature with retentate flow rate of 1400-1600ml/min and permeate flow rate of 65-70ml/min.
Samples were kept frozen until filtration. Prior to filtration, samples were thawed at 37°C.

**Starting materials and filtration buffers**

To evaluate the effect of the macromolecule on the nanofiltration, various starting materials were prepared and the filtration buffers were adjusted accordingly.

**Table 1. Starting materials and their filtration buffers**

| Sample name | Starting material | Molecule added | Filtration buffer |
|---|---|---|---|
| THa | Thrombin in-process sample with mannitol 20mg/ml | Human albumin (Albutein, Alpha) 2mg/ml | Mannitol 20mg/ml Sodium acetate tri hydrate 2.72mg/ml Human albumin 2mg/ml |
| THaI | As above | Human albumin (Albutein, Alpha 1mg/ml | Mannitol 20mg/ml Sodium acetate tri hydrate 2.72mg/ml Human albumin 1mg/ml |
| THb | As above | Human albumin 0.2mg/ml | Mannitol 20mg/ml Sodium acetate tri hydrate 2.72mg/ml Human albumin 0.2mg/ml |
| THbI | As above | Human albumin 0.01mg/ml | Mannitol 20mg/ml Sodium acetate tri hydrate 2.72mg/ml Human albumin 0.01mg/ml |
| THc | As above | Human albumin 0.01mg/ml | Mannitol 20mg/ml Sodium acetate tri hydrate 2.72mg/ml |
| THe | As above | Dextran 80kDa (Fluka) 0.23mg/ml | Mannitol 20mg/ml Sodium acetate tri hydrate 2.72mg/ml Dextran 80kDa (Fluka) 0.23mg/ml |

(continued)

| Sample name | Starting material | Molecule added | Filtration buffer |
|---|---|---|---|
| THf | As above | Dextran 25kDa (Fluka) 0.07mg/ml | Mannitol 20mg/ml Sodium acetate tri hydrate 2.72mg/ml Dextran 25kDa (Fluka) 0.07mg/ml |
| THk | As above | Dextran 5kDa (Fluka) 0.015mg/ml | Mannitol 20mg/ml Sodium acetate tri hydrate 2.72mg/ml Dextran 5kDa (Fluka) 0.015mg/ml |
| THi | NaCl 14.6 mg/ml Sodium acetate trihydrate 2.72mg/ml mannitol | No molecule added | Mannitol 20mg/ml Sodium acetate tri hydrate 2.72mg/ml |
| DMEM | DMEM (Biological Industries | No molecule added | DMEM |

**Tangential flow nanofiltration (TFNF) using Viresolve70™ module.**

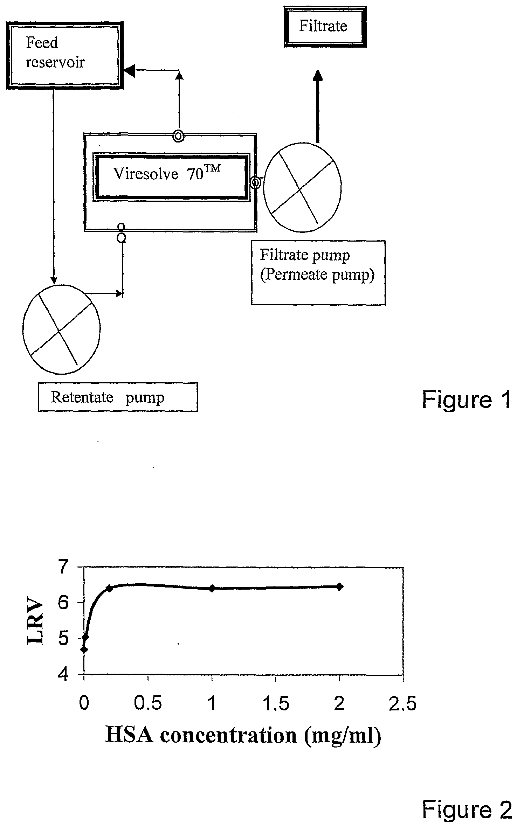

The production size Viresolve 70™ module contains a nominal membrane area of $0.1m^2$. In this study, scaled down modules containing 1/3 or 1/6 of the production membrane area were used and the conditions were adjusted accordingly. The tangential flow nanofiltartion was carried out according to the manufacturer's recommendations. Briefly, Viresolve 70™ filtration process included the use of 2 peristaltic pumps: one (retenteate) was used to circulate the product through the module and the other (permeate) was used to drag out the filtrate and collect it (Fig. 1). In the case of 1/6 scaled down ratio, the pumps were pre-adjusted to achieve the desired flow rates of $250\pm10$ ml/min for the retentate pump and 11-12ml/min for the permeate pump. When the 1/3 scaled down ratio was used, the flow rates were doubled. Prior to product filtration the module was washed with purified water and then with filtration buffer. The content of the filtration buffer was determined by the starting material used (see Table 1).

1-1.5 liters of starting material to be filtered was used with the 1/6 scaled down module and 2-3 liters was used with the 1/3 scaled down module. Prior to Viresolve 70™ filtration, samples were filtered through a $0.2\mu m$ filter (Millipak 40, Millipore). The filtration process was carried out at room temperature using the flow rates described above. At the end of starting material filtration the Viresolve module was subjected to 3 washes with the relevant filtration buffer followed by a cleaning procedure which included filtrate and retentae washing with purified water. Samples collected during the process were used for the determination of MVMp or HAV titer, and protein and thrombin concentration (Table 2).

**Table 2. Sample collected**

| Sample name | Description | Purpose |
|---|---|---|
| N2-negative control | Sample was collected from pre-spiked starting material either undiluted or 2 fold diluted and stored at -70°C | 1. Undiluted sample was used for thrombin and protein/Dextran concentration<br>2. Two fold diluted (in culture medium) served as negative control when added to A9 cell culture. |
| V1, V2 positive control | Samples were collected from MVMp stock used for the study.<br>**V1** sample were diluted 10 fold in culture medium and stored at -70°C<br>**V2** samples were held for the duration of the experiment at room temperature, diluted 10 fold in culture medium and stored at -70°C | **V1-**was used as a reference for the virus stock titer used in this study and for the evaluation of the starting material and experiment condition effects on MVM(p) titer<br>**V2-**was used to evaluate experiment condition effect on MVM(p) titer |

(continued)

| Sample name | Description | Purpose |
|---|---|---|
| Pre-filtered L1,L2 | Samples were collected from the starting material post spiking. **L1** samples were diluted 10 fold in culture medium and stored at -70°C **L2** samples were held for the duration of the experiment at room temperature, diluted 10 fold in culture medium and stored at -70°C | **L1** was used as a reference for the evaluation of experiment condition effect on MVM(p) titer **L2** was used to evaluate experiment condition effect on MVM(p) titer when the virus was in the starting material |
| F-filtrate | Samples collected at the end of the filtration process from the filtrate were either undiluted or 2 fold diluted and stored at -70°C | 1.Undiluted was used for thrombin and protein/Dextran concentration 2. Two fold diluted were used for MVMp titration and cytotoxicity and interference ( from the relevant runs) |
| R-retentate | Samples were collected at the end of the filtration and washing process, from the solution left at the feed reservoir, diluted 2 fold in culture medium and stored at -70°C | **R** samples were used for MVM(p) titration |
| W-washing | Samples were collected at the end of the washing procedure from the washing filtrate, diluted 2 fold in culture medium and stored at -70°C | **W** samples were used for MVM(p) titration |
| CR-retentate cleaning | Samples were removed at the end of the retentate track cleaning procedure from the entire volume passed through the retentate track. Samples were diluted 2 fold in culture medium and stored at -70°C | **CR** samples were used for MVM(p) titration |
| CF-filtrate cleaning | Samples were removed at the end of the permeate track cleaning procedure from the entire filtrate volume. Samples were diluted 2 fold in culture medium and stored at -70°C | **CF** samples were used for MVM(p) titration |

**Results**

To assess the effect of macromolecule addition on small non-enveloped virus removal and product recovery through Viresolve filtration, a series of tangential nanofiltration experiments were conducted. As a model, thrombin purification was investigated using a Viresolve 70™ module. The first part of the study was designed to optimize the operating conditions for the Viresolve 70™ (1/3 ft$^2$) system using the manufacturer's flux excursion protocol. The experiment was carried out as follows: on the day of the experiment a thrombin solution was thawed at 37°C and then filtered through a 500 cm$^2$ 0.2 $\mu$m polyethersulfone filter (CH5925PPZK, PALL). The feed container was then filled with 300 ml of filtered thrombin solution. The retentate pump flow rate was set to 500 ml/min, and the product was recirculated over the membrane for 30 min. Then, the permeate pump was set to achieve flow rates of 3.5, 5, 10, 15, 20, 25 and 30 ml/min and operated for each flow rate with the permeate being returned to the retentate line for 30 min (total recirculation). Samples of retentate (R) and permeate (P) were collected for each flow rate and stored at -70°C.

The membrane was then cleaned according to Millipore operating procedures.

The frozen samples were thawed and tested for their protein concentration and thrombin activities.

The experiment was repeated twice, the first time after cleaning the membrane and the second time using a new membrane. Each time, the runs were performed with a new thrombin vial.

The optimal flow rate was chosen according to the highest protein recovery and thrombin measurement.

An integrity test was performed on the Viresolve 70 module according to the manufacturer's instructions.

The results obtained indicated (see Tables 3 and 4) that the best permeate flow rate was 20-25 ml/min. At these flow rates, the average thrombin and protein recoveries were approximately 93% and 75%, respectively.

**Table 3. The effect of permeate flow rate on total protein recovery**

| Flow rate permeate(ml/min) | sample | Protein mg/ml | | | Protein recovery (%) | | | Average | SD |
|---|---|---|---|---|---|---|---|---|---|
| | | run 1 | run 2 | run 3 | run 1 | run 2 | run 3 | protein recovery | protein recovery |
| 3.5 | R₁ | 1.918 | 1.915 | 1.968 | | | | | |
| 3.5 | P₁ | 1.207 | 0.732 | 1.090 | 62.93 | 38.22 | 55.37 | 52.18 | 12.66 |
| 5 | R₂ | 1.942 | 1.853 | 2.024 | | | | | |
| 5 | P₂ | 1.081 | 0.725 | 1.099 | 55.66 | 39.13 | 54.29 | 49.69 | 9.18 |
| 10 | R₃ | 2.167 | 1.906 | 2.094 | | | | | |
| 10 | P₃ | 1.386 | 1.114 | 1.237 | 63.96 | 58.45 | 59.10 | 60.50 | 3.01 |
| 15 | R₄ | 1.998 | 1.739 | 2.111 | | | | | |
| 15 | P₄ | 1.442 | 1.24 | 1.378 | 72.17 | 71.31 | 65.26 | 69.58 | 3.76 |
| 20 | R₅ | 1.996 | 1.505 | 2.142 | | | | | |
| 20 | P₅ | 1.613 | 1.271 | 1.278 | 80.81 | 84.45 | 59.69 | 74.98 | 13.37 |
| 25 | R₆ | 1.849 | 1.699 | 2.012 | | | | | |
| 25 | P₆ | 1.509 | 1.28 | 1.399 | 81.61 | 75.34 | 69.54 | 75.50 | 6.04 |
| 30 | R₇ | 1.52 | 2.011 | 1.873 | | | | | |
| 30 | P₇ | 0.99 | 1.089 | 1.222 | 65.13 | 54.15 | 65.23 | 61.50 | 6.37 |

**Table 4. The effect of permeate flow rate on thrombin recovery**

| Flow rate permeate (ml/min) | sample | Thrombin IU/ml | | | Thrombin recovery (%) | | | Average | SD |
|---|---|---|---|---|---|---|---|---|---|
| | | run 1 | run 2 | run 3 | run 1 | run 2 | run 3 | Thrombin recovery | Thrombin recovery |
| 3.5 | R₁ | 770 | 670 | 795 | | | | | |
| 3.5 | P₁ | 690 | 520 | 675 | 89.61 | 77.61 | 84.9 | 84.04 | 6.05 |
| 5 | R₂ | 800 | 650 | 790 | | | | | |
| 5 | P₂ | 690 | 595 | 655 | 86.25 | 91.54 | 82.9 | 86.90 | 4.35 |
| 10 | R₃ | 770 | 610 | 790 | | | | | |
| 10 | P₃ | 730 | 530 | 685 | 94.81 | 86.89 | 86.7 | 89.47 | 4.62 |
| 15 | R₄ | 820 | 650 | 770 | | | | | |
| 15 | P₄ | 730 | 570 | 685 | 89.02 | 87.69 | 89.0 | 88.56 | 0.75 |
| 20 | R₅ | 800 | 660 | 765 | | | | | |
| 20 | P₅ | 750 | 580 | 720 | 93.75 | 87.88 | 94.1 | 91.92 | 3.50 |
| 25 | R₆ | 770 | 670 | 755 | | | | | |
| 25 | P₆ | 770 | 570 | 730 | 100.0 | 85.07 | 96.7 | 93.92 | 7.84 |
| 30 | R₇ | 860 | 625 | 795 | | | | | |
| 30 | P₇ | 810 | 605 | 720 | 94.19 | 96.80 | 90.6 | 93.85 | 3.13 |

Once the optimum flow rate was determined, thrombin filtration using production scale-down conditions could be evaluated. For these experiments, the feed container was filled with 3000 ml thrombin solution and samples ($T_0$) were taken.

The recirculation pump was set to flow rate of 500 ml/min and the product was recirculated for 30 min. The batch volume was processed at the optimal flux determined by the flux excursion experiment until the hold-up volume was reached. Samples were collected from the feed container ($C_1$) and the permeate ($P_1$). The solution was diafiltered with the appropriate buffer at 3x the hold-up volume to recover as much protein mass as possible without significant product dilution, and a further permeate sample ($P_2$) was collected.

The protein concentration and thrombin activity in the samples before and after diafiltration were measured to determine product recovery with and without diafiltration. The Viresolve 70 module was kept at 4°C and an integrity test was performed according to the manufacturer's instructions for testing the membrane's integrity.

The results obtained (Tables 5 and 6) indicated that the mean thrombin recovery was about 96%, and the mean protein recovery was about 77%. In addition, the diafiltration step (W) resulted in the recovery of an additional 3-5% of thrombin activity.

**Table 5. Protein recovery post Viresolve 70 (1/3 ft$^2$) filtration using production scale-down conditions**

| | | Sample: | | | Volume (ml) | | | Total protein (mg) | | | Recovery (%) | Average ±SD (%) |
|---|---|---|---|---|---|---|---|---|---|---|---|---|
| | | $T_0$ | F | W | $T_0$ | F | W | $T_0$ | F | W | | |
| Protein (mg/ml) | run # 1 | 1.582 | 1.643 | 1.302 | 2914 | 2900 | 236 | 4609.9 | 4764.7 | 307.272 | 103.3 | **77.2±27.6** |
| | run # 2 | 1.59 | 1.312 | 1.474 | 3000 | 2900 | 235 | 4770 | 3804.8 | 346.4 | 79.8 | |
| | run # 3 | 1.893 | 0.941 | 1.959 | 3080 | 3000 | 245 | 5830.4 | 2823 | 479.95 | 48.4 | |

**Table 6. Thrombin recovery post Viresolve 70 (1/3 ft$^2$) filtration using production scale-down conditions**

| | | Sample: | | | Volume (ml) | | | Total Thrombin (IU) | | | Recovery (%) | Average ±SD (%) |
|---|---|---|---|---|---|---|---|---|---|---|---|---|
| | | $T_0$ | F | W | $T_0$ | F | W | $T_0$ | F | W | | |
| **Thrombin IU/m** | run#1 | 725 | 720 | 276 | 2914 | 2900 | 236 | 2112650 | 2088000 | 65136 | 101.9 | |
| | run#2 | 845 | 750 | 498 | 3000 | 2900 | 235 | 2535000 | 2175000 | 117030 | 90.4 | **96.1±5.7** |
| | run#3 | 990 | 927 | 579 | 3080 | 3000 | 245 | 3049200 | 2781000 | 141855 | 95.9 | |

Once the conditions for thrombin tangential flow using a Viresolve 70™ were set, the efficiency of the system to remove viruses could be studied.

In the first experiments described below, the optimal flux excursion was used. The nominal membrane area of the modules used was 1/3 ft or 1/6 ft. Therefore the volume of the product to be filtered and the flow rates were adjusted accordingly.

In all the experiments conducted, the thrombin in-process samples to be filtered, were spiked with MVM(p) or HAV to a final concentration of more than $1\times10^5$ $TCID_{50}$ units/ml (for details see Methods section). The spiked solutions were filtered through a $0.2\mu m$ filter and the first pre filtration samples (L1 and L2) were removed for viral titration. The remaining spiked material was filtered through a pre-equilibrated Viresolve 70™ membrane using the relevant filtration buffer. The filtration process proceeded until retentate volume reached 100-250ml. The entire filtrate was collected and sample was removed for viral titration (F). The filtration process continued with repeated washing of the filter using the relevant filtration buffer. The filtrate of the washing step was either collected separately and additional sample was collected for virus titration (W), or combined with the previous filtrate (virus titers in these samples were calculated accordingly). The retentate from the entire process was collected at that stage, and sample (R) was removed for virus titration Post filtration, all the modules were washed and tested for integrity by the integrity test specified by the module manufacturer (Millipore).

**Table 7. Removal of parvovirus from thrombin solution using Viresolve70, under optimal condition determined by the flux excursion experiment**

| Thrombin solution +HSA (2mg/ml)+ Mannitol 20mg/ml | | |
|---|---|---|
| Sample | MVM(p) titter (log) | |
| | Run 1 | Run 2 |
| Pre-filtered (L2) | 10.19 | 10.08 |
| Filtrate (F)+washing (W) | 3.0 | 4.29 |
| Retentate (R) | 9.45 | 9.46 |
| log L2-log F | 7.19 | 5.78 |

The results obtained (shown in Table 7) demonstrated that under optimal conditions, in regards to thrombin and protein recoveries, the removal of parvoviruses using this system is highly efficient. Moreover the results obtained were unexpectedly better than reported in previous studies (Hoffer et al 1995, DiLeo et al 1992, DiLeo et al 1993, Jernberg et al 1996, Adamson, 1998).

In order to validate these results and to clarify the mechanism for the improved reduction results, a series of experiments were conducted.

The first set of experiments was designed to evaluate the contribution of albumin concentration to virus removal. Four concentrations of albumin solutions were used: 2mg/ml, 1mg/ml, 0.2mg/ml and 0.01 mg/ml, supplemented with 20mg/ml mannitol. These solutions were spiked with either HAV or MVM(p) using the conditions and processes described above. However, during the experiment with MVM, the flow rate was maintained at the rate fixed at the beginning of the filtration (worst case conditions; increase pressure during filtration). Conversely for HAV, the flow rate was fixed at the beginning of the filtration and the filtration was then carried out without an increase of pressure. The results obtained demonstrated that indeed the reduction values obtained previously for MVM(p) of about 6.0 log reduction (LRV) were repeatable (see Table 8 and Fig. 2). Interestingly, addition of HSA at a concentration of 0.2-2 mg/ml showed a similar effect on MVM(p) removal values. However, addition of HSA at a 20-fold lower concentration (0.01mg/ml) or omitting albumin from the starting material resulted in a substantially lower removal of MVM(p) (about 5.0 or 4.7 log reduction, respectively), indicating that macromolecules such as albumin have a role in the improved reduction factor. Of note was the thrombin molarity in the various albumin solutions of $5.4\mu M$. These results suggest that in order to improve the removal of MVM(p) it is sufficient to use a lower molarity of HSA as compared to the molarity of thrombin.

The reduction values for HAV in general were higher (>7.0LRV) (Table 9) since HAV is a larger virus (27-32nm) as compared to MVM(p) (18-26nm). As shown for MVM, HSA at concentrations of 0.2-2mg/ml had a similar effect on the removal factor of HAV. Furthermore, the increased pressure used to maintain the initial flow rate in the runs that included MVM, did not reduce virus removal.

**Table 8. The effect of various HSA concentration solutions on MVM(p) removal through Viresolve 70 filtration**
(Indicated are the log titers of MVM(p))

| Sample | HSA 2.0mg/ml (=31μM) | HSA 1.0mg/ml (=15μM) | HSA 0.2mg/ml (=3.1μM) | HSA 0.01mg/ml (=0.15μM) | No HSA |
|---|---|---|---|---|---|
| Pre-filtered (L2) | 10.06 | 9.95 | 9.80 | 9.77 | 9.49 |
| Filtrate (F) | 3.78 | 3.53 | 3.40 | 4.74 | 4.80 |
| Retentate | 9.55 | 9.29 | 9.32 | 8.74 | 8.75 |
| log L2-log F | 6.46 | 6.41 | 6.40 | 5.03 | 4.69 |

**Table 9. The effect of various HSA concentration solutions on HAV (HM 175) removal by Viresolve 70 filtration.**
(Indicated are log titers of HAV)

| Sample | HSA 2.0mg/ml (=31μM) | HSA 1.0mg/ml (=15μM) | HSA 0.2mg/ml (=3.1μM) |
|---|---|---|---|
| Pre-filtered (L2) | 9.35 | 9.45 | 9.75 |
| Filtrate (F) | <2.03 | 3.38 | <2.03 |
| Retentate | 9.20 | 8.89 | 8.72 |
| log L2-log F | >7.32 | 6.07 | >7.71 |

To further test the involvement of albumin in the removal process, the next set of experiments was conducted. The effect of the following solutions on virus removal was examined: thrombin solution containing 0.2mg/ml albumin and 20 mg/ml mannitol, mannitol solution and DMEM medium. The conditions used for the process and the spiking procedure were as described above.

The results obtained (shown in Table 10) showed that a further reduction in the virus removal value was obtained when thrombin was omitted in addition to the omission of HSA (2.84 LRV), while the lowest reduction value was obtained when DMEM medium (Biological Industries) was used (1.72 LRV).

**Table 10. The effect of various starting material composition on MVMp reduction using Viresolve TFNF**

| Sample | DMEM | No Thr No HSA + Man | + Thr + HSA (0.2mg/ml) + Man |
|---|---|---|---|
| Pre-filtered (L2) | 9.41 | 9.22 | 9.43 |
| Filtrate (F) | 7.69 | 6.38 | 3.50 |
| Retentate (R) | 9.85 | 9.57 | 8.05 |
| log L2-log F | 1.72 | 2.84 | 5.93 |

To assess the effect of the addition of other macromolecules on virus removal and whether molecular size has any effect on virus removal, dextran solutions of various molecular sizes with the same molarity were used. These molecules were added to an in-process sample as described above. The spiking and filtration process was performed according to the condition described above.

The results obtained (Table 11) showed that in the first experiment, where 80kDa dextran at a final concentration of 0.23mg/ml was used, about 6.2 log reduction in MVM(p) titer was found. A similar effect on viral removal values was found when smaller sized dextran molecules (5kDa, 25kDa) were used with the same molar ratio, and MVM(p) titer post filtration was reduced again by about 6.1-6.2 logs.

**Table 11. The effect of starting material solutions supplemented with various molecular size Dextran on MVM (p) reduction using Viresolve TFNF**

| Sample | MVM(p) titer (log) | | |
|---|---|---|---|
| | + Dex 5kDa + Thr + Man | + Dex 25kDa + Thr + Man | + Dex 80kDa + Thr + Manl |
| Pre-filtered (L2) | 9.17 | 8.42 | 8.80 |

(continued)

| Sample | MVM(p) titer (log) | | |
|---|---|---|---|
| | + Dex 5kDa + Thr + Man | + Dex 25kDa + Thr + Man | + Dex 80kDa + Thr + Manl |
| Filtrate (F) | 3.03 | 2.25 | 2.55 |
| Retentate (R) | 8.43 | 7.49 | 7.76 |
| log L2-log F | 6.14 | 6.17 | 6.25 |

To assess whether increased flow rate would affect virus removal values, the permeate flow rate was increased to 25ml/min and maintained throughout the filtration process. The results showed that the increased flow rate had only a marginal effect on MVM(p) removal values (Table 12) and a reduction of about 5.7 log was obtained.

**Table 12. The influence of higher permeate flow rate (25ml/min) on MVM(p) clearance**

| Sample | HSA (2mg/ml)+ Man | |
|---|---|---|
| | MVM(p) titter (log) | |
| | Run 1 | Run2 |
| Pre-filtered (L2) | 9.81 | 9.34 |
| Filtrate (F) | 4.03 | 3.59 |
| Retentate | 8.38 | 8.96 |
| log L2-log F | 5.78 | 5.74 |

In addition, when the average flow rates of various filtrations runs were measured (see Table 13) it was found that the flow rate was reduced dramatically in solutions containing a macromolecule (i.e. albumin or dextran) in addition to the thrombin. The lowest average flow rate of 5.4ml/min, which is about 46% of the initial flow rate was reached when thrombin solution was supplemented with human albumin to a final concentration of 2.0mg/ml. Between 63% to about 74% of the initial flow rate was reached when the final concentration of albumin was 0.2mg/ml or when equimolar concentrations of various sized dextran molecules were used. These differences in the average flow rate had no effect on virus removal values as described in previous experiments. However, when no macromolecule was added to the thrombin solution or when only buffers (e.g DMEM, buffer + mannitol) were used, the average flow rate was close to the initial flow rate.

**Table 13. The effect of Thrombin solution content on filtration flow rate**

| Run | Volume (ml) | Time (min) | Average Flow rate (ml/min) | % of the initial flow rate** |
|---|---|---|---|---|
| Scale down (HSA 2mg/ml+mannitol 20mg/ml) | 1212.8 | 223 | 5.4 | 46 |
| Th04+mannitol 20mg/ml+HSA 0.2mg/ml | 1182.1 | 139 | 8.5 | 72 |
| Th04+mannitol 20mg/ml Dex 80kDa (0.23mg/ml) | 1071.5 | 138 | 7.8 | 66 |
| Th04+mannitol 20mg/ml Dex 25kDa (0.07mg/ml) | 1063.9 | 143 | 7.4 | 63 |
| Th04+mannitol 20mg/ml Dex 5kDa (0.014mg/ml) | 1103.7 | 126 | 8.7 | 74 |
| Th04+mannitol 20mg/ml | 1575.2 | 147 | 10.7 | 91 |
| DMEM* | 1920.1 | 98 | 19.6 | 87 |

(continued)

| Run | Volume (ml) | Time (min) | Average Flow rate (ml/min) | % of the initial flow rate** |
|---|---|---|---|---|
| Buffer (+mannitol)* | 1879.4 | 93 | 20.2 | 90 |

*The 1/3 ft filter was used
** When the 1/3ft filter was used the flow rate was set to 22.5ml/min When the 1/6ft filter was used the flow rate was set to 11.7ml/min

## Claims

1. A method for enhancing infective particle removal from a thrombin solution comprising:

   (a) adding to said solution a macromolecule of at least 3 monomers selected from sugars and sugar alcohols, and
   (b) passing said solution through a nanofilter,
   thereby enhancing infective particle removal from the thrombin solution, wherein the log reduction value (LRV) of the infective particles after step (b) is above 4, and wherein the macromolecule is not a non-ionic surfactant.

2. The method according to claim 1 wherein said solution originates from whole blood or plasma.

3. The method according to claim 1, wherein the recovery of the purified thrombin after step (b) is in the range of 59-100% or in the range of 75-93%.

4. The method according to claim 1, wherein the LRV of the particles after step (b) is up to 8.

5. The method according to claim 1, wherein the particles are in the size range of 15 to 80 nm or larger than 80 nm.

6. The method according to any one of claims 1 to 5 wherein said macromolecule is selected from the group consisting of a polymer of at least 6 monomers.

7. The method of any one of claims 1 to 6 wherein said macromolecule is selected from the group consisting polysaccharides such as dextran molecules; cellulose derivatives; vinyl polymers and glycols.

8. The method according to any one of claims 1 to 7 wherein said macromolecule is added within a concentration range of more than 0.01 mg/ml to less than or equal to 2 mg/ml; more than 0.15 $\mu$M to less than or equal to 31 $\mu$M or at a concentration that is lower than the concentration of the thrombin solution.

9. The method according to any one of claims 1 to 8 wherein said nanofilter has a particle size cutoff of $>10^4$ Dalton or of about $10^4$-$3\times10^5$ Dalton such as a Viresolve/70™ or a Viresolve/180™ membrane.

10. The method according to any one of claims 1 to 9 wherein the solution is passed through the nanofilter by tangential flow nanofiltration (TFNF) or by dead-end nanofiltration.

## Patentansprüche

1. Verfahren zum Verbessern der Entfernung infektiöser Partikel aus einer Thrombinlösung, umfassend:

   (a) Zugeben eines Makromoleküls aus mindestens 3 Monomeren, ausgewählt aus Zucker und Zuckeralkoholen, zu der Lösung und
   (b) Durchleiten der Lösung durch einen Nanofilter,
   wodurch die Entfernung infektiöser Partikel aus der Thrombinlösung verbessert wird, wobei der logarithmische Reduktionswert (LRV) der infektiösen Partikel nach Schritt (b) über 4 liegt, und wobei das Makromolekül kein nichtionisches Tensid ist.

2. Verfahren gemäß Anspruch 1, wobei die Lösung aus Vollblut oder Plasma stammt.

3. Verfahren gemäß Anspruch 1, wobei die Rückgewinnung des gereinigten Thrombins nach Schritt (b) im Bereich von 59-100 % oder im Bereich von 75-93 % liegt.

4. Verfahren gemäß Anspruch 1, wobei der LRV der Partikel nach Schritt (b) bis zu 8 beträgt.

5. Verfahren gemäß Anspruch 1, wobei die Partikel im Größenbereich von 15 bis 80 nm oder größer als 80 nm liegen.

6. Verfahren gemäß einem der Ansprüche 1 bis 5, wobei das Makromolekül ausgewählt ist aus der Gruppe bestehend aus einem Polymer aus mindestens 6 Monomeren.

7. Verfahren gemäß einem der Ansprüche 1 bis 6, wobei das Makromolekül ausgewählt ist aus der Gruppe bestehend aus Polysacchariden wie zum Beispiel Dextranmolekülen, Cellulosederivaten, Vinylpolymeren und Glykolen.

8. Verfahren gemäß einem der Ansprüche 1 bis 7, wobei das Makromolekül in einem Konzentrationsbereich von mehr als 0,01 mg/ml bis weniger als oder gleich 2 mg/ml, mehr als 0,15 $\mu$M bis weniger als oder gleich 31 $\mu$M oder in einer Konzentration, die niedriger ist als die Konzentration der Thrombinlösung, zugegeben wird.

9. Verfahren gemäß einem der Ansprüche 1 bis 8, wobei der Nanofilter eine Partikelgrößenbegrenzung von >$10^4$ Dalton oder von etwa $10^4$-$3 \times 10^5$ Dalton aufweist, wie zum Beispiel eine Viresolve/70™- oder eine Viresolve/180™-Membran.

10. Verfahren gemäß einem der Ansprüche 1 bis 9, bei die Lösung durch den Nanofilter durch Tangentialfluss-Nanofiltration (TFNF) oder durch Sack-Nanofiltration geleitet wird.

## Revendications

1. Procédé pour augmenter l'élimination de particules infectieuses d'une solution de thrombine comprenant :

   (a) l'ajout, à ladite solution, d'une macromolécule d'au moins 3 monomères choisis parmi des sucres et des alcools de sucre, et
   (b) le passage de ladite solution à travers un nanofiltre,

   ce qui permet d'augmenter l'élimination de particules infectieuses de la solution de thrombine, dans lequel la valeur de réduction logarithmique (LRV) des particules infectieuses après l'étape (b) est supérieure à 4, et dans lequel la macromolécule n'est pas un tensioactif non ionique.

2. Procédé selon la revendication 1 dans lequel ladite solution provient de sang entier ou de plasma.

3. Procédé selon la revendication 1, dans lequel la récupération de la thrombine purifiée après l'étape (b) est dans la plage de 59 à 100 % ou dans la plage de 75 à 93 %.

4. Procédé selon la revendication 1, dans lequel la LRV des particules après l'étape (b) va jusqu'à 8.

5. Procédé selon la revendication 1, dans lequel les particules sont dans la plage de tailles de 15 à 80 nm ou plus grosses que 80 nm.

6. Procédé selon l'une quelconque des revendications 1 à 5 dans lequel ladite macromolécule est choisie dans le groupe constitué par un polymère d'au moins 6 monomères.

7. Procédé selon l'une quelconque des revendications 1 à 6 dans lequel ladite macromolécule est choisie dans le groupe constitué par des polysaccharides tels que des molécules de dextrane ; des dérivés de cellulose ; des polymères vinyliques et des glycols.

8. Procédé selon l'une quelconque des revendications 1 à 7 dans lequel ladite macromolécule est ajoutée dans une plage de concentration de plus de 0,01 mg/ml à 2 mg/ml ou moins ; de plus de 0,15 $\mu$M à 31 $\mu$M ou moins ou à une concentration qui est inférieure à la concentration de la solution de thrombine.

9. Procédé selon l'une quelconque des revendications 1 à 8 dans lequel ledit nanofiltre a un seuil d'exclusion de taille de particule supérieure à $10^4$ Dalton ou d'environ $10^4$ à $3 \times 10^5$ Dalton comme une membrane Viresolve/70™ ou Viresolve/180™.

10. Procédé selon l'une quelconque des revendications 1 à 9 dans lequel on fait passer la solution à travers le nanofiltre par nanofiltration à flux tangentiel (TFNF) ou par nanofiltration frontale.

Figure 1

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0131740 A
- US 4481189 A
- WO 9600237 A, Winge
- US 6096872 A, Van Holten
- US 6773600 B, Rosenblatt

### Non-patent literature cited in the description

- **DILEO, A.J. et al.** *Biotechnology,* 1992, vol. 10, 182-188
- **DILEO, A.J. et al.** *Biologicals,* 1993, vol. 21, 275-286
- **HOFFER, L. et al.** *J. Chromatography,* 1995, vol. 669, 187-196
- **WALLACE R.E.** *In vitro,* 1973, vol. 8, 333-341